# EUROPEAN PATENT SPECIFICATION

(11) **EP 0 480 572 B1**
(45) Date of publication and mention of the grant of the patent: **07.06.1995**
(21) Application number: 91307807.7
(22) Date of filing: 23.08.1991
(51) Int. Cl.: A61K 7/48, A61K 31/59, C07C 401/00

(54) **Use of vitamin D compounds for the manufacture of a cosmetic composition and use of it for improvements of skin conditions**
Verwendung von Vitamin D Verbindungen zur Herstellung einer kosmetischen Zusammensetzung und Anwendung dieser zur Verbesserung der Hautbeschaffenheit
Utilisation des composés de vitamine D pour la fabrication d'un composé cosmétique et son utilisation pour améliorer la condition de la peau

(30) Priority: 24.08.1990 US 573560; 24.08.1990 US 573339; 24.08.1990 US 573634; 24.08.1990 US 572857
(43) Date of publication of application: 15.04.1992
(73) Proprietor: WISCONSIN ALUMNI RESEARCH FOUNDATION, Madison, WI 53705 (US)
(72) Inventor: DeLuca, Hector Floyd, Deerfield, Wisconsin 53531 (US); Smith, Connie Marie, Madison, Wisconsin 53713 (US)
(74) Representative: Ellis-Jones, Patrick George Armine

(56) References cited:
- EP-A- 0 129 003
- EP-A- 0 387 077
- WO-A-89/10351
- US-A- 4 800 198
- US-A- 4 853 378
- Steroides, vol.26, no.4, 422-436, (1975);
- Biochemistry, 1990, 29, 190-196.

## Description

The present invention relates to cosmetic compositions, and more particularly to such compositions containing vitamin D compounds.

Skin problems range between severe skin disorders such as dermatitis, eczema, psoriasis, solar keratosis and the like, and less severe skin conditions such as wrinkles, lack of dermal hydration i.e. dry skin, lack of adequate skin firmness i.e. skin slackness, insufficient sebum secretion and the like. The former skin disorders have typically been treated with compositions termed "dermatological" whereas the latter skin conditions have typically been treated with compositions termed "cosmetic" since the primary functions of such compositions are to preserve, condition or protect the skin.

In the past, treatment of various skin disorders and skin conditions has been largely based on non-specific-drugs. For example, dermatitis has been commonly treated with corticosteroids. Such compounds may provide symptomatic relief for some patients. However, steroids are known to produce numerous local and systemic side effects, and their long term use is not recommended.

Holick et al U. S. Patent No. 4,728,643 discloses a method of treating psoriasis by administering to a patient a vitamin D compound capable of differentiating cultured tumor cells. Examples of such compounds are vitamins D₂ or D₃ or derivatives of vitamins D₂ or D₃.

Dikstein et al U. S. Patent No. 4,610,978 and U. S. Reissue Patent No. 33,107 disclose cosmetic and dermatological compositions containing 1 alpha-hydroxycholecalciferol or 1 alpha, 25-dihydroxycholecalciferol. These compositions are disclosed for use in the topical treatment of skin disorders and skin conditions such as dermatitis, psoriasis, eczema, solar keratosis, wrinkles, dry skin and skin slackness.

Japanese published patent application No. 62/169711 entitled "A Skin Cosmetic Material" discloses a skin cosmetic composition containing vitamin D₃ or vitamin D₃ derivatives. The vitamin D₃ derivatives disclosed include 25-hydroxycholecalciferol, 1 alpha-hydroxycholecalciferol, 5,6-trans-25-hydroxycholecalciferol, 1 alpha-25-dihydroxycholecalciferol and dihydrotachysterol.

Finally, DeLuca et al U. S. Patent No. 4,800,198 discloses the structure of the secosterol compounds of interest in the present compositions. However, the patent is directed toward a method for inducing the differentiation of malignant cells utilizing secosterol compounds, and in particular to the treatment of leukemoid diseases with such compounds. The patent does not teach or suggest any cosmetic uses for the secosterol compounds disclosed therein.

Compositions containing one or more vitamin D compounds selected from 1α-hydroxyvitamin D homolog compounds, 19-nor-vitamin D compounds and secosterol compounds, as defined below together with a suitable carrier useful in the treatment of various cosmetic skin conditions are described.

Various formulations for the compositions are also provided. Such formulations may include creams, lotions, ointments, and the like. The compositions and/or formulations may also include additional active ingredients if desired.

The compounds disclosed herein unexpectedly provide highly effective treatments for the skin conditions such as wrinkles, lack of dermal hydration i.e. dry skin, lack of adequate skin firmness i.e. slack skin, and insufficient sebum secretion without producing unwanted systemic or local side effects.
Fig. 1 is a photomicrograph at X200 magnification of a skin replica of a control mouse treated topically with a propylene glycol control vehicle;
Fig. 2 is a photomicrograph at X200 magnification of a skin replica of a second control mouse treated intraperitoneally with a propylene glycol control vehicle;
Fig. 3 is a photomicrograph at X200 magnification of a skin replica of an experimental mouse treated topically with Δ²², 24, 24, 24-trihomo-1α,25-dihydroxyvitamin D₃;
Fig. 4 is a photomicrograph at X200 magnification of a skin replica of a second experimental mouse treated topically with Δ²² 24, 24, 24-trihomo-1α,25-dihydroxyvitamin D₃;
Fig. 5 is a photomicrograph at X200 magnification of a skin replica of an experimental mouse treated intraperitoneally with Δ²², 24, 24, 24-trihomo-1α,25-dihydroxyvitamin D₃;
Fig. 6 is a photomicrograph at X200 magnification of a skin replica of a second experimental mouse treated intraperitoneally with Δ²², 24, 24, 24-trihomo-1α,25-dihydroxy vitamin D₃;
Fig. 7 is a photomicrograph at X200 magnification of a skin replica of an experimental mouse treated topically with 1α-hydroxy-bis-homo-pregnacalciferol;
Fig. 8 is a photomicrograph at X200 magnification of a skin replica of a second experimental mouse treated topically with 1α-hydroxy-bis-homo-pregnacalciferol;
Fig. 9 is a photomicrograph at X200 magnification of a skin replica of an experimental mouse treated intraperitoneally with 1α-hydroxy-bis-homo-pregnacalciferol;
Fig. 10 is a photomicrograph at X200 magnification of a skin replica of a second experimental mouse treated intraperitoneally with 1α-hydroxy-bis-homo-pregnacalciferol.
Fig. 11 is a photomicrograph at X2000 magnification of a skin replica of an experimental mouse treated topically with 1α,25-dihydroxy-19-nor-vitamin D₃;
Fig. 12 is a photomicrograph at X1000 magnification of a skin replica of a second experimental mouse treated topically with 1α,25-dihydroxy-19-nor-vitamin D₃;
Fig. 13 is a photomicrograph at X2000 magnification of a skin replica of an experimental mouse treated intraperitoneally with 1α,25-dihydroxy-19-nor-vitamin D₃;
Fig. 14 is a photomicrograph at X1000 magnification of a skin replica of a second experimental mouse treated intraperitoneally with 1α,25-dihydroxy-19-nor-vitamin D₃.

It has been found, according to the present invention, that effective treatment of various skin conditions can be achieved with a vitamin D compound selected from 1α-hydroxyvitamin D homolog compounds, 19-nor vitamin D compounds and secosterol compounds, defined below.

The 1α-hydroxyvitamin D homolog compounds useful in the compositions of the present invention are characterized structurally as side chain unsaturated and side chain saturated homologs of vitamin D, and preferably of 1,25-(OH)₂D₃ in which the side chain is elongated by insertion of one or more methylene units into the chain at the carbon 24 position. They may be represented, therefore, by the following general structure of formula I: 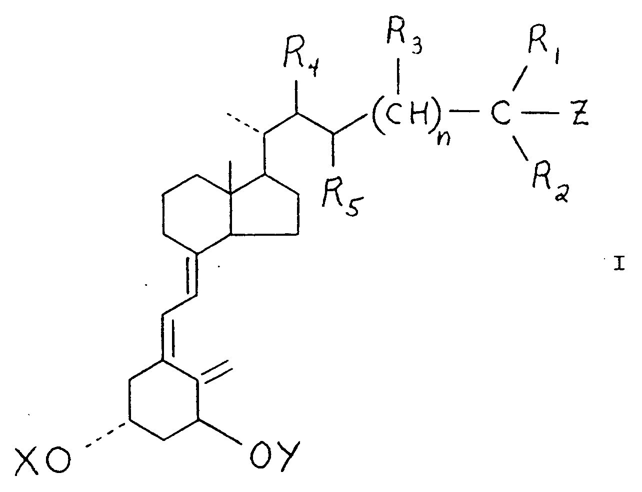
where R₄ and R₅ represent hydrogen or when taken together R₄ and R₅ represent a carbon-carbon single bond or a carbon-carbon double bond, Z represents hydrogen, hydroxy or protected-hydroxy, R₃ represents hydroxy, protected hydroxy or an alkyl group, X and Y which may be the same or different are hydrogen or a hydroxy-protecting group, R₁ represents the group -(CH₂)_{q}-H and R₂ represents the group -(CH₂)ₚ-H, and where n, q and p are integers having independently the values of 1 to 5 with the proviso that at least one of n, q and p is greater than 1, and R₁ and R₂ when taken together represent the group -(CH₂)ₘ- where m is an integer having the value of 2 to 5.

US-A-4 853 378 discloses 26,26,26,27,27,27-hexafluoro vitamins D₃ derivatives which have a fluorine atom at the 23 and/or 24 carbon atom, which are said to be useful in treating psoriasis. WO 89/10351 discloses vitamin D₃ compounds for use in treating psoriasis.

The 19-nor-vitamin D compounds referred to herein are a class of 1α-hydroxylated vitamin D compounds in which the ring A exocyclic methylene group (carbon 19) typical of all vitamin D systems has been removed and replaced by two hydrogen atoms. Structurally these novel analogs are characterized by the general formula II shown below: 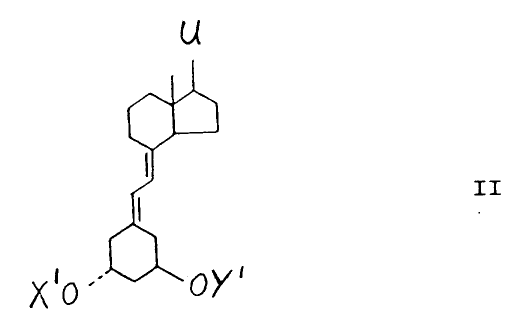
where X¹ and Y¹ are each selected from the group consisting of hydrogen, acyl, alkylsilyl and alkoxyalkyl, and where U represents an alkyl, hydrogen, hydroxyalkyl or fluoroalkyl group, or the following side chain: 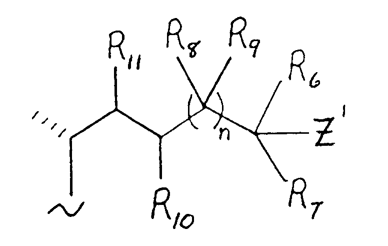
wherein Z¹ represents hydrogen, hydroxy or O-acyl, R₆ and R₇ are each selected from the group consisting of alkyl, hydroxyalkyl and fluoroalkyl, or, when taken together represent the group -- (CH₂)ₘ -- where m is an integer having a value of from 2 to 5, R₈ is selected from the group consisting of hydrogen, hydroxy, fluorine, O-acyl, alkyl, hydroxyalkyl and fluoroalkyl, R₉ is selected from the group consisting of hydrogen, fluorine, alkyl, hydroxyalkyl and fluoroalkyl, or, R₈ and R₉ taken together represent double-bonded oxygen or double-bonded carbon, R₁₀ and R₁₁ are each selected from the group consisting of hydrogen, hydroxy, O-acyl, fluorine and alkyl, or, R₁₀ and R₁₁ taken together form a carbon-carbon single bond or a carbon-carbon double bond, and wherein n is an integer having a value of from 1 to 5, and wherein the carbon at any one of positions 20, 22, or 23 in the side chain may be replaced by an O, S, or N atom.

Specific important examples of side chains for the 19-nor compounds are the structures represented by formulas (a), (b), (c), (d) and (e) below, i.e. the side chain as it occurs in 25-hydroxyvitamin D₃ (a); vitamin D₃ (b); 25-hydroxyvitamin D₂ (c); vitamin D₂ (d); and the C-24-epimer of 25-hydroxyvitamin D₂ (e). 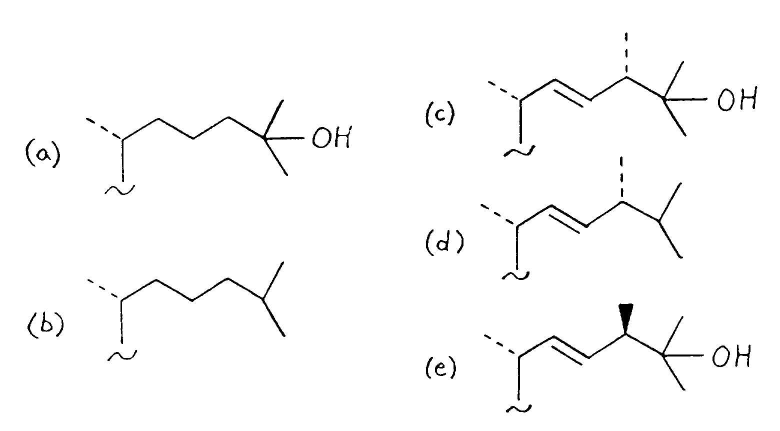
EP-A-0 387 077, which forms part of the state of the art under Art.54(3) EPC discloses such 19-nor compounds for use in the treatment of psoriasis and other skin conditions characterised by proliferation of undifferentiated skin cells.

Purely structurally, the class of secosterol compounds referred to herein has a similarity with some of the known vitamin D compounds. Unlike the known vitamin D compounds, however, the secosterols used in the present invention do not express the classic vitamin D activities in vivo, i.e. stimulation of intestinal calcium transport, or the mobilization of bone calcium, and hence they cannot be classified as vitamin D derivatives from the functional point of view. In light of the prior art, it was all the more surprising and unexpected then, to find that these secosterols are remarkably effective in the treatment of skin conditions. This finding provides an effective method for the treatment of skin conditions, since the above described secosterols can be administered to subjects in doses sufficient to treat the skin condition, without producing simultaneously unphysiologically high and deleterious blood calcium levels.

The group of secosterols exhibiting this unique and heretofore unrecognized activity pattern is characterized by the general structure III shown below: 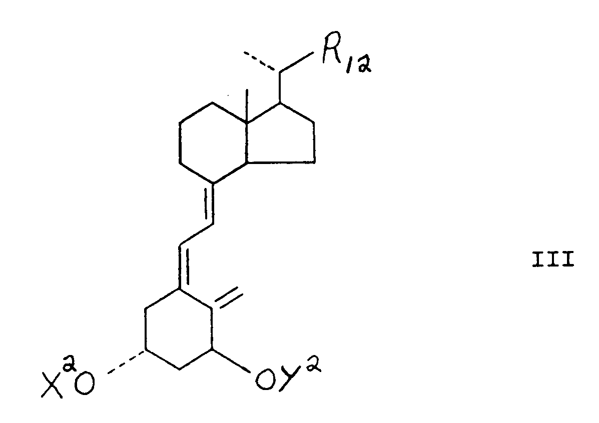
where R₁₂ is hydrogen, methyl, ethyl or propyl and where each of X² and Y² represent, independently, hydrogen, an acyl group, or a hydroxy-protecting group.

As used in the description, and in the claims, the term "hydroxy-protecting group" refers to any group commonly used for the protection of hydroxy functions during subsequent reactions, including, for example, acyl or alkylsilyl groups such as trimethylsilyl, triethylsilyl, t-butyldimethylsilyl and analogous alkylated silyl radicals, or alkoxyalkyl groups such as methoxymethyl, ethoxymethyl, methoxyethoxymethyl, tetrahydrofuranyl or tetrahydropyranyl. A "protected-hydroxy" is a hydroxy function derivatized by one of the above hydroxy-protecting groupings. "Alkyl" represents a straight-chain or branched hydrocarbon radical of 1 to 10 carbons in all its isomeric forms, such as methyl, ethyl, propyl, isopropyl, butyl, isobutyl, pentyl, etc., and the terms "hydroxyalkyl" and "fluoroalkyl" refer to such an alkyl radical substituted by one or more hydroxy or fluoro groups respectively. An acyl group is an alkanoyl group of 1 to 6 carbons in all its isomeric forms, or an aroyl group, such as benzoyl, or halo-, nitro- or alkyl-substituted benzoyl groups, or a dicarboxylic acyl group such as oxalyl, malonyl, succinoyl, glutaroyl, or adipoyl. The term "aryl" signifies a phenyl-, or an alkyl-, nitro- or halo-substituted phenyl group.

It should be noted in this description that the term "24-dihomo" refers to the addition of two methylene groups at the carbon 24 position in the side chain. Likewise, the term "trihomo" refers to the addition of three methylene groups. Also, the term "26,27-dimethyl" refers to the addition of a methyl group at the carbon 26 and 27 positions so that for example R₁ and R₂ are ethyl groups. Likewise, the term "26,27-diethyl" refers to the addition of an ethyl group at the 26 and 27 positions so that R₁ and R₂ are propyl groups.

Specific and preferred examples of these compounds when the side chain is unsaturated (i.e. R₄ and R₅ represent a double bond) are: 24-dihomo-1,25-dihydroxy-22-dehydrovitamin D₃, i.e. the compound shown above, where X and Y are hydrogen, Z is hydroxy, n equals 3, and R₁ and R₂ are each a methyl group; 26,27-dimethyl-24-dihomo-1,25-dihydroxy-22-dehydrovitamin D₃, i.e. the compound shown above where X and Y are hydrogen, Z is hydroxy, n equals 3, and R₁ and R₂ are each an ethyl group; 24-trihomo-1,25-dihydroxy-22-dehydrovitamin D₃, i.e. the compound having the structure shown above, where X and Y are hydrogen, Z is hydroxy, n equals 4, and R₁ and R₂ are each a methyl group; 26,27-dimethyl-24-trihomo-1,25-dihydroxy-22-dehydrovitamin D₃, i.e. the compound shown above where X and Y are hydrogen, Z is hydroxy, n equals 4, and R₁ and R₂ are each an ethyl group; 26,27-diethyl-24-dihomo-1,25-dihydroxy-22-dehydrovitamin D₃, i.e. the compound shown above where X and Y are hydrogen, Z is hydroxy, n equals 3, and R₁ and R₂ are each a propyl group; 26,27-diethyl-24-trihomo-1,25-dihydroxy-22-dehydrovitamin D₃, i.e. the compound shown above where X and Y are hydrogen, Z is hydroxy, n equals 4, and R₁ and R₂ are each a propyl group, 26,27-dipropyl-24-dihomo-1,25-dihydroxy-22-dehydrovitamin D₃, i.e. the compound shown above where X and Y are hydrogen, Z is hydroxy, n equals 3, and R₁ and R₂ are each a butyl group; and 26,27-dipropyl-24-trihomo-1,25-dihydroxy-22-dehydrovitamin D₃, i.e. the compound shown above where X and Y are hydrogen, Z is hydroxy, n equals 4, and R₁ and R₂ are each a butyl group.

Specific and preferred examples of these compounds when the side chain is saturated (i.e. R₄ and R₅ each represent hydrogen) are: 24-dihomo-1,25-dihydroxy-vitamin D₃, i.e. the compound shown above, where X and Y are hydrogen, Z is hydroxy, n equals 3, and R₁ and R₂ are each a methyl group; 26,27-dimethyl-24-dihomo-1,25-dihydroxy-vitamin D₃, i.e. the compound shown above where X and Y are hydrogen, Z is hydroxy, n equals 3, and R₁ and R₂ are each an ethyl group; 24-trihomo-1, 25-dihydroxy-vitamin D₃, i.e. the compound having the structure shown above, where X and Y are hydrogen, Z is hydroxy, n equals 4, and R₁ and R₂ are each a methyl group; 26,27-dimethyl-24-trihomo-1,25-dihydroxy-vitamin D₃, the compound shown above where X and Y are hydrogen, Z is hydroxy, n equals 4, and R₁ and R₂ are each an ethyl group; 26,27-diethyl-24-dihomo-1,25-dihydroxy-vitamin D₃, i.e. the compound shown above where X and Y are hydrogen, Z is hydroxy, n equals 3, and R₁ and R₂ are each a propyl group; 26, 27-diethyl-24-trihomo-1,25-dihydroxy-vitamin D₃, i.e. the compound shown above where X and Y are hydrogen, Z is hydroxy, n equals 4, and R₁ and R₂ are each a propyl group; 26,27-dipropyl-24-dihomo-1,25-dihydroxy-vitamin D₃, i.e. the compound shown above where X and Y are hydrogen, Z is hydroxy, n equals 3, and R₁ and R₂ are each a butyl group; and 26,27-dipropyl-24-trihomo-1,25-dihydroxy-vitamin D₃, i.e. the compound shown above where X and Y are hydrogen, Z is hydroxy, n equals 4, and R₁ and R₂ are each a butyl group.

### Preparation of Homologated Saturated And Unsaturated Side Chain Compounds:

Examples of the compounds used in this invention wherein the side chain is saturated can be prepared according to the general process described in U. S. Patent No. 4,927,815. Examples of the compounds wherein the side chain is unsaturated can be prepared according to the general process illustrated and described in U. S. Patent No. 4,847,012. Examples of the compounds wherein R₁ and R₂ together represent a cyclopentano group can be prepared according to the general process described in U. S. Patent No. 4,851,401.

Another synthetic strategy for the preparation of side-chain-modified analogues of 1α,25-dihydroxyergocalciferol is disclosed in Kutner et al, The Journal of Organic Chemistry, 1988, Vol. 53, pages 3450-3457. In addition, the preparation of 24-homo and 26-homo vitamin D analogs are disclosed in U. S. Patent No. 4,717,721 issued January 5, 1988 entitled "Sidechain Homo-Vitamin D Compounds With Preferential Anti-Cancer Activity" the description of which is specifically incorporated herein by reference.

### Preparation of 19-Nor-Vitamin D Compounds

The preparation of 1α-hydroxy-19-nor-vitamin D compounds having the basic structure shown above in formula II can be accomplished by a common general method, using known vitamin D compounds as starting materials. For the synthesis of 1α,25-dihydroxy-19-nor-vitamin D₃, reference is made to Perlman et al, Tetrahedron Letters, 1990, Vol. 31, No. 13, pages 1823-1824. Suitable starting materials are, for example, the vitamin D compounds of the general structure IV: 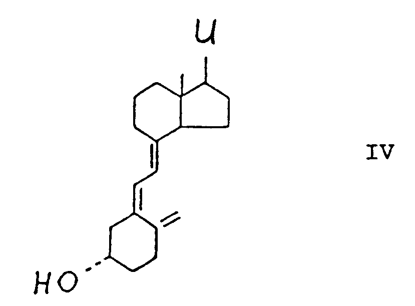
where U is any of the side chains as defined above. These vitamin D starting materials are known compounds, or compounds that can be prepared by known methods.

Using the procedure of DeLuca et al U.S. Patent 4,195,027, the starting material is converted to the corresponding 1α-hydroxy-3,5-cyclovitamin D derivative, having the general structure V below, where X³ represents hydrogen and Q represents an alkyl, preferably methyl: 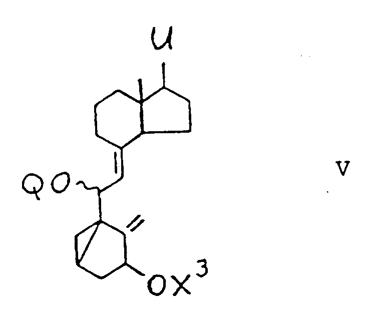
So as to preclude undesired reaction of the 1α-hydroxy group in subsequent steps, the hydroxy group is converted to the corresponding acyl derivative, i.e. the compound V shown above, where X³ represents an acyl group, using standard acylation procedures, such as treatment with an acyl anhydride or acyl halide in pyridine at room temperature or slightly elevated temperature (30-70°C). It should be understood also that whereas the process of this invention is illustrated here with acyl protection of hydroxy functions, alternative standard hydroxy-protecting groups can also be used, such as, for example, alkylsilyl or alkoxyalkyl groups. Such protecting groups are well-known in the art (e.g. trimethylsilyl, triethylsilyl, t.-butyldimethylsilyl, or tetrahydrofuranyl, methoxymethyl), and their use is considered a routine modification of experimental detail within the scope of the process of this invention.

The derivative as obtained above is then reacted with osmium tetroxide, to produce the 10,19-dihydroxy analog, VI (where X³ is acyl), which is subjected to diol cleavage using sodium metaperiodate or similar vicinal diol cleavage reagents (e.g. lead tetraacetate) to obtain the 10-oxo-intermediate, having the structure VII below (where X³ is acyl): 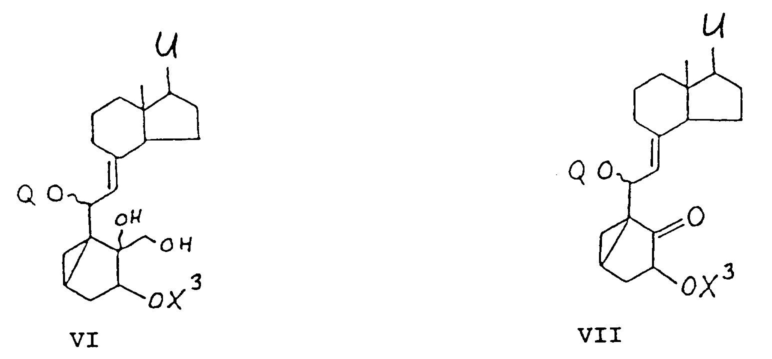
These two consecutive steps can be carried out according to the procedures given by Paaren et al. (J. Org. Chem. 48, 3819 (1983)). If the side chain unit, U carries vicinal diols (e.g. 24,25-dihydroxy- or 25,26-dihydroxy, etc.), these, of course, also need to be protected, e.g. via acylation, silylation, or as the isopropylidene derivative prior to the periodate cleavage reactions.

In most cases, the acylation of the 1α-hydroxy group as mentioned above will simultaneously effect the acylation of side chain hydroxy functions, and these acylation conditions can, of course, be appropriately adjusted (e.g. elevated temperatures, longer reaction times) so as to assure complete protection of side chain vicinal diol groupings.

The next step of the process comprises the reduction of the 10-oxo-group to the corresponding 10-alcohol having the structure VIII shown below (where X³ is acyl and Y³ represents hydroxy). When X³ is acyl, this reduction is carried out conveniently in an organic solvent at from about 0°C to about room temperature, using NaBH₄ or equivalent hydride reducing agents, selective for the reduction of carbonyl groups without cleaving ester functions. Obviously, when X³ is a hydroxy-protecting group that is stable to reducing agents, any of the other hydride reducing agents (e.g. LiAlH₄, or analogous reagents) may be employed also. 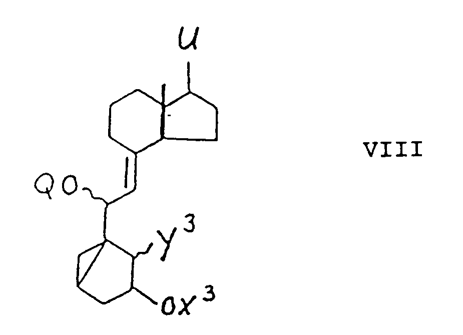
The 10-hydroxy intermediate is then treated with an alkyl-or arylsulfonylhalide (e.g. methanesulfonylchloride) in a suitable solvent (e.g. pyridine) to obtain the corresponding 10-0-alkyl-or arylsulfonyl derivative (the compound having the structure shown VIII above, where Y³ is alkyl-SO₂O-, or aryl-SO₂O-, and this sulfonate intermediate is then directly reduced, with lithium aluminum hydride, or the analogous known lithium aluminum alkyl hydride reagents in an ether solvent, at a temperature ranging from O°C to the boiling temperature of the solvent, thereby displacing the sulfonate group and obtaining the 10-deoxy derivative, represented by the structure VIII above, where X³ and Y³ are both hydrogen. As shown by the above structure, a 1-0-acyl function in the precursor compound VII is also cleaved in this reduction step to produce the free 1α-hydroxy function, and any 0-acyl protecting group in the side chain would, of course, likewise be reduced to the corresponding free alcohol function, as is well understood in the art. If desired, the hydroxy groups at C-1 (or hydroxy groups in the side chain) can be reprotected by acylation or silylation or ether formation to the corresponding acyl, alkylsilyl or alkoxyalkyl derivative, but such protection is not required. Alternative hydroxy-protecting groups, such as alkylsilyl or alkoxyalkyl groups would be retained in this reduction step, but can be removed, as desired, at this or later stages in the process by standard methods known in the art.

The above 1α-hydroxy-10-deoxy cyclovitamin D intermediate is next solvolyzed in the presence of a low-molecular weight organic acid, using the conditions of DeLuca et al U.S. Patents 4,195,027 and 4,260,549. When the solvolysis is carried out in acetic acid, for example, there is obtained a mixture of 1α-hydroxy-19-nor-vitamin D 3-acetate and 1α-hydroxy-19-nor-vitamin D 1-acetate (compounds IX and X, below), and the analogous 1- and 3-acylates are produced, when alternative acids are used for solvolysis. 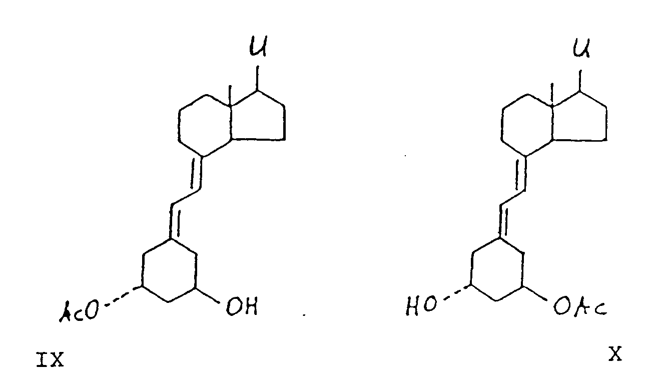
Direct basic hydrolysis of this mixture under standard conditions then produces the desired 1α-hydroxy-19-nor-vitamin D compounds of structure II above (where X¹ and Y¹ are both hydrogen). Alternatively, the above mixture of monoacetates may also be separated (e.g. by high pressure liquid chromatography) and the resulting 1-acetate and 3-acetate isomers may be subjected separately to hydroxysis to obtain the same final product from each, namely the 1α-hydroxy-19-nor-vitamin D compounds of structure II. Also the separated monoacetates of structure IX or X or the free 1,3-dihydroxy compound can, of course, be reacylated according to standard procedures with any desired acyl group, so as to produce the product of structure II above, where X¹ and Y¹ represent acyl groups which may be the same or different.

### Preparation of Secosterol Compounds:

The secosterol of structure III where R₁₂ is hydrogen can be prepared according to the method of Lam et al as published in Steroids 26, 422 (1975), the description of which is specifically incorporated herein by reference. The secosterols of structure III, where R₁₂ is methyl, ethyl or propyl, can be prepared according to the general process illustrated and described in U. S. Patent No. 4,800,198 issued January 24, 1989 entitled "Method of Inducing the Differentiation of Malignant Cells With Secosterol", the description of which is specifically incorporated herein by reference.

In accordance with the present invention, the above side chain saturated and unsaturated 1α-hydroxyvitamin D homolog compounds together with the above 19-nor-vitamin D compounds and secosterol compounds are employed in compositions, formulations thereof and methods of using the same for the treatment of such skin conditions as dry skin (lack of dermal hydration), undue skin slackness (i.e., insufficient skin firmness) and insufficient sebum secretion. The compositions are also effective in the general preservation, conditioning and protecting of the skin, e.g., against wrinkles.

Compositions for use in the above-mentioned treatment of skin comprise an effective amount of one or more side chain unsaturated or side chain saturated 1α-hydroxyvitamin D homolog compound, one or more 19-nor-vitamin D compound, or one or more secosterol compound as the active ingredient, and a suitable carrier. An "effective amount" of such compounds is typically from about 0.001 »g to about 10.0 »g per gm of composition, and may generally be administered orally or parenterally in dosages of from about 0.1 »g/day to about 25 »g/day. A concentration of 0.01 »g per gm of the composition is preferred.

The compositions may be formulated as a foam (which may contain a propellant), a stick, a cleansing pad, an impregnated wipe, a face pack, a shaving foam or an after shave, but preferably as creams, lotions or ointments by choice of appropriate carriers. Suitable carriers include vegetable or mineral oils, white petrolatum (white soft paraffin), branched chain fats or oils, animal fats and high molecular weight alcohol (greater than C₁₂). The preferred carriers are those in which the active ingredient is soluble. Thickening agents (so that the composition is in the form of an ointment, cream, lotion or gel), other active cosmetic ingredients including anti-wrinkle agents and anti-grease agents along with additives such as surfactants, soaps, bath additives, organic solvents, emulsifiers, stabilizers and antioxidants may also be included as well as agents imparting color or fragrance if desired.

Creams are preferably formulated from a mixture of mineral oil, self-emulsifying beeswax and water in which mixture the active ingredient, dissolved in a small amount of an oil such as almond oil, is admixed. A typical example of such a cream is one which includes about 40 parts water, about 20 parts beeswax, about 40 parts mineral oil and about 1 part almond oil.

Ointments may be formulated by mixing a solution of the active ingredient in a vegetable oil such as almond oil with warm soft paraffin and allowing the mixture to cool. A typical example of such an ointment is one which includes about 30% almond oil and about 70% white soft paraffin by weight.

Lotions may be conveniently prepared by dissolving the active ingredient, in a suitable high molecular weight alcohol such as propylene glycol or polyethylene glycol.

One or more additional substances which have therapeutic effects on the skin may also be incorporated in the compositions. Thus in one embodiment of this invention the composition also contains one or more compounds capable of increasing cyclic-AMP levels in the skin. Suitable compounds include adenosine or a nucleic acid hydrolysate, e.g. in an amount of about 0.1-1% and papaverine, e.g. in an amount of about 0.5-5%, both by weight based on the weight of the composition. Also suitable are β-adrenergic agonists such as isoproterenol, e.g. in an amount of about 0.1-2% or cyclic-AMP, e.g. in an amount of about 0.1-1%, again both by weight based on the weight of the composition. Other suitable types of additional active ingredients which may be incorporated in the compositions of this invention include other compounds known to have a beneficial effect on skin. Such compounds include retinoids such as Vitamin A, e.g. in an amount of about 0.003%-0.3% by weight and chromanols such as Vitamin E or a derivative thereof, e.g. in an amount of about 0.1-10% by weight, both based on the weight of the composition. Additionally, anti-inflammatory agents and keratoplastic agents may be incorporated in the cosmetic composition. A typical anti-inflammatory agent is a corticosteroid such as hydrocortisone or its acetate, e.g. in an amount of about 0.25-5% by weight, or a corticosteroid such as dexamethasone, e.g. in an amount of about 0.025-0.5% by weight, both based on the weight of the composition. A typical keratoplastic agent is coal tar, e.g. in an amount of about 0.1-20% or anthralin, e.g. in an amount of about 0.05-2% by weight, both based on the weight of the composition.

Topical application, intraperitoneal injection (parenteral) and oral administration of compositions of this invention was found to be cosmetically effective in field studies. In a typical example, topical application of a lotion containing 0.01 »g of a 1α-hydroxyvitamin D homolog compound per gram of lotion to the skin of nude mice for five weeks resulted in improved skin condition.

### Cosmetic Efficacy of 1α-hydroxyvitamin D Compounds:

The cosmetic efficacy of the 1α-hydroxyvitamin D homolog compounds used in this invention was determined by the following procedures:
Two treatment groups of six mice each were available with Group I being controls and Group II behind the treatment group with Trihomo-D₃. Three mice in each group received the treatment topically (t) and three mice received it intraperitoneally (ip) three times a week for about 5 weeks. By visual evaluation, the mice treated with the Trihomo-D₃ compound (b) had the pinkest, smoothest skin as compared to the control mice.

Replicas were made about 48 hours after the last treatment of the backs of 5 control animals and 4 experimental animals. Silflo™ silicone rubber was spread onto the rear half of each mouse back (anesthetized with diethyl ether) and allowed to polymerize for 5 to 7 minutes. These silicone rubber "negative" replicas were stored in glassine envelopes until polyethylene "positive" replicas were made. The procedures for preparing both the negative and the positive replicas will hereinafter be described.

The nine positive replicas were coated with about 60 nm gold and examined in a JEOL JSM-35C scanning electron microscope at 15 kV accelerating voltage. Differences between replicas were evident to the unaided eye and from Polaroid micrographs made of each replica at X12 to form a montage of the entire surface. Micrographs were also made at X100 and X200 to differentiate fine details of skin surface condition.

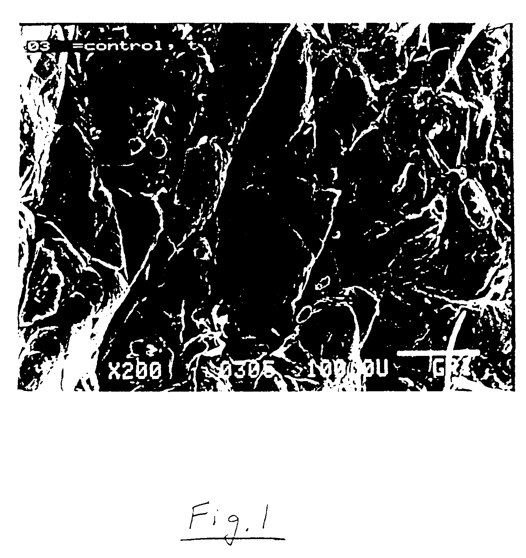

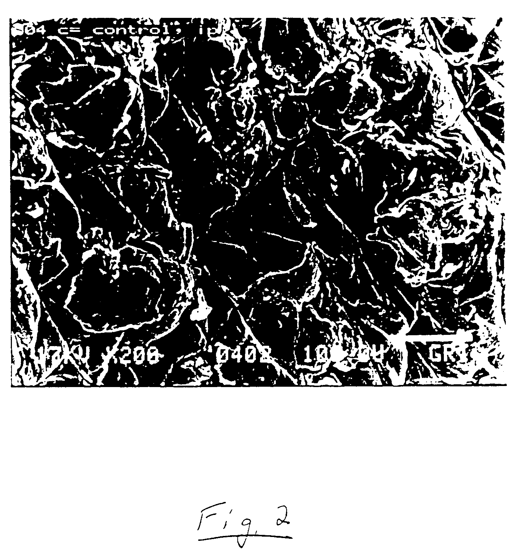

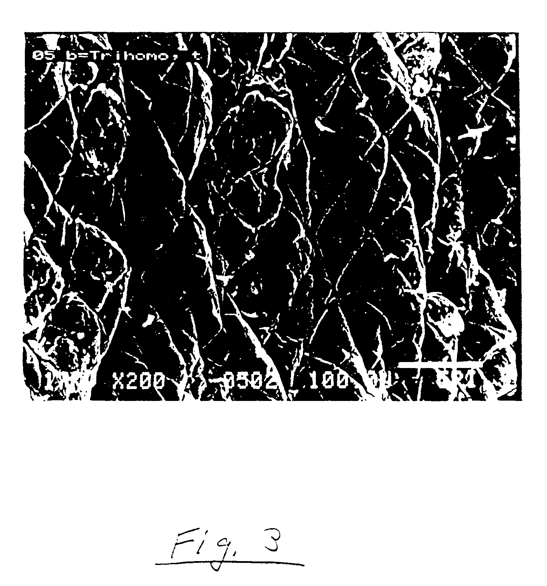

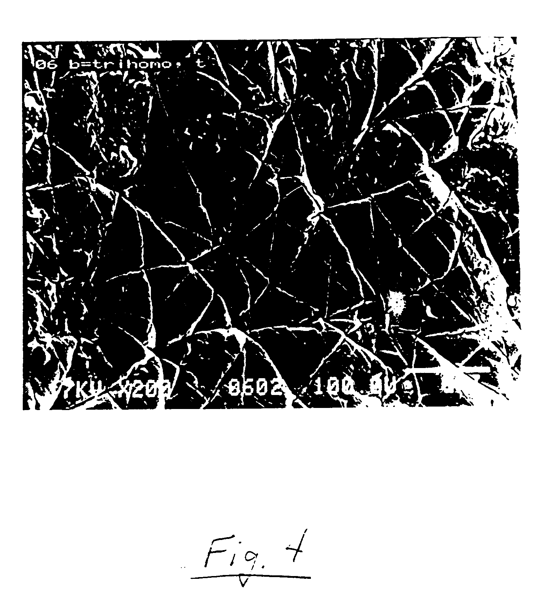

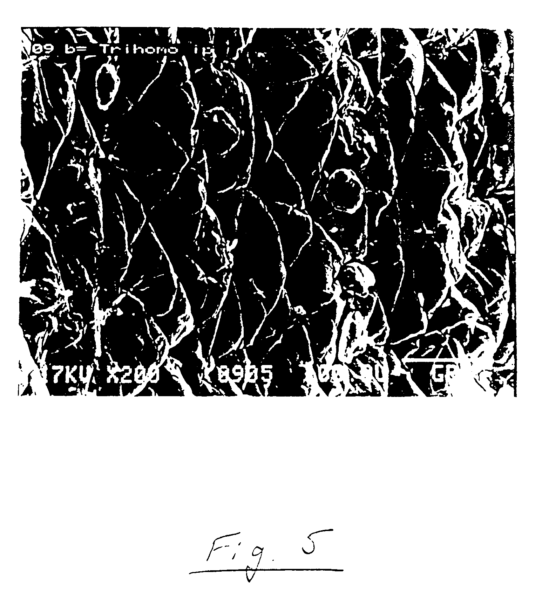

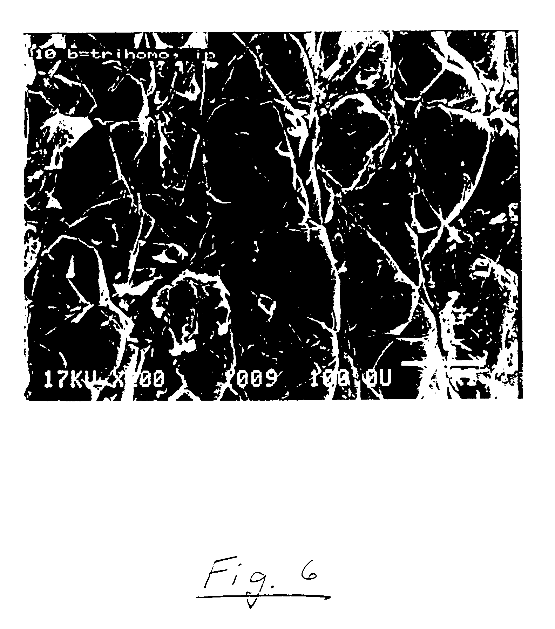

Figures 1 and 2 illustrate the skin surface condition of two of the control mice treated topically and intraperitoneally with a propylene glycol vehicle only. Figures 3 and 4 illustrate the skin condition of two of the experimental mice treated topically with Δ²², 24, 24, 24-trihomo-1α,25-dihydroxyvitamin D₃ (Trihomo D₃), whereas Figures 5 and 6 illustrate the skin condition of two of the experimental mice treated intraperitoneally with Δ²², 24,24,24-trihomo-1α,25-dihydroxyvitamin D₃.

The results of the above experiments were that the low magnification (X12) montages of the nine skin replicas could be ranked into four groups according to visible wrinkling and overall skin surface roughness. The best skin condition (rank 1) was exhibited by samples #5, #6, and #8, all of which were treated with Trihomo-D₃. The next best was #7. The third rank included three of the control samples. The remaining two controls could be placed alone in a fourth rank having the roughest skin (Table 1).

Details of the skin surface such as stratum corneum desquamation, scaling, size and plumpness of skin divisions, were visible on higher magnification (X100 and X200) micrographs and used to rank the replicas from 1=best skin to 6=worst skin (Tables 1 and 2). The treated samples ranked 1 to 4, and the five controls ranked 5 and 6. The top-ranked four included all of the Trihomo-D₃ samples (#5, #6, #7, #8). There was no discernible difference between topical and intraperitoneal Trihomo-D₃, i.e. #6, #7, #8 were very nearly equal in appearance.

As a result of these experiments, it may be concluded that topical and intraperitoneal treatments of nude mice with 1α-hydroxy Vitamin D analogs produce visible changes in skin surface condition as seen on replicas. Treated samples showed less gross wrinkling and scaling and more smooth, plump, rounded skin microtopography than the respective controls. Treatment with Trihomo-D₃ clearly had a greater effect than no treatment at all. Topical treatment could not be differentiated from intraperitoneal treatment.

The visible changes in skin surface condition (as shown in Figs. 3-6 versus Figs. 1-2), is in marked contrast with that of compositions containing ergocalciferol or cholecalciferol. Topical application of compositions containing ergocalciferol, for instance, were of low cosmetic efficacy and in fact resulted in decreased skin elasticity (See Table I in Dikstein et al U. S. Reissue 33,107). Moreover, since it is known that ergocalciferol and cholecalciferol are absorbed into the bloodstream through the skin, it is likely that doses of such compounds applied to large areas of the skin or applied chronically, even in the minimal active dose, cause systemic effects. Further, the compounds of the present invention demonstrated no expected or observed side effects.

**TABLE 1**

| SEM OF MOUSE SKIN REPLICAS | | | |
|---|---|---|---|
| No. | Treatment | Rank*(X12) | Rank*(X100,200) |
| #1 | c-t control | 3 | 6 |
| #2 | c-t control | 4 | 6 |
| #3 | c-t control | 3 | 6 |
| #4 | c-ip control | 3 | 6 |
| #5 | b-t Trihomo-D₃ | 1 | 4 |
| #6 | b-t Trihomo-D₃ | 1 | 1 |
| #7 | b-ip Trihomo-D₃ | 2 | 2 |
| #8 | b-ip Trihomo-D₃ | 1 | 3 |
| #9 | c-ip control | 4 | 5 |

| | | | |
|---|---|---|---|
| *Scanning electron micrographs at X100 and X200 were judged for skin condition and ranked, with 1=best (smoothest, plumpest, least scaling) and 6=worst (roughest, most scaling). | | | |
| Treatments: b=Trihomo-D₃ | | | |
| c=Control, vehicle only | | | |
| t=topical, 160 ng Trihomo-D₃/20»l propylene glycol applied 3 times per week | | | |
| ip=intraperitoneal, 50ng Trihomo-D₃/50»l propylene glycol given 3 times per week | | | |

**TABLE 2**

| RANKING OF MOUSE SKIN REPLICAS | | |
|---|---|---|
| No. | Treatment | Rank@(X12) |
| 1 | #6 b-t | 1 |
| 2 | #7 b-ip | 2 |
| 3 | #8 b-ip | 1 |
| 4 | #5 b-t | 1 |
| 5 | #9 c-ip | 4 |
| 6 | #1 c-t | 3 |
| 6 | #2 c-t | 4 |
| 6 | #3 c-t | 3 |
| 6 | #4 c-ip | 3 |

| | | |
|---|---|---|
| *Scanning electron micrographs at X100 and X200 were judged for skin condition and ranked, with 1=best (smoothest, plumpest, least scaling) and 6=worst (roughest, most scaling). | | |
| @Photo montages at X12 magnification were made of each 25mm diameter replica and ranked for skin condition, with 1=best (smoothest) and 4=worst (roughest, more wrinkled). | | |
| Treatments: b=Trihomo-D₃ | | |
| c=Control, vehicle only | | |
| t=topical | | |
| ip=intraperitoneal | | |

### Cosmetic Efficacy of 19-nor-vitamin D Compounds:

Topical application and intraperitoneal injection of cosmetic compositions of 19-nor-vitamin D compounds used in this invention was found to be cosmetically effective in field studies. In a typical example, topical application of a lotion containing 0.01 »g of a 19-nor-vitamin D compound per gram of lotion to the skin of nude mice three times per week for five weeks resulted in improved skin condition.

The cosmetic efficacy of compositions containing 19-nor-vitamin D compounds used in this invention was determined by the following procedures:

Two treatment groups of seven mice each were available with one group being controls, and the other group being the treatment group with 1α,25-dihydroxy-19-nor-vitamin D₃. The mice in the control group were further defined as Group 1 which contained four mice treated topically (t) only with a propylene glycol vehicle, and Group 2 which contained three mice treated intraperitoneally (ip) only with a propylene glycol vehicle three times a week for 5 weeks. The mice in the treatment group were further defined as Group 3 which contained three mice treated topically (t) with 19-nor-1α,25-dihydroxy-vitamin D₃, and Group 4 which contained four mice treated intraperitoneally (ip) with 19-nor-1α,25-dihydroxy-vitamin D₃ three times a week for five weeks.

Replicas were made about 48 hours after the last treatment of the backs of two mice from each group, i.e. four control animals and four experimental animals. Silflo™ silicone rubber was spread onto the rear half of each mouse back (anesthetized with diethyl ether) and allowed to polymerize for 5 to 7 minutes. These silicone rubber "negative" replicas were stored in glassine envelopes until polyethylene "positive" replicas were made. The procedures for preparing both the negative and positive replicas will hereinafter be described.

The eight positive replicas were coated with about 60 nm gold and examined in a JEOL JSM-35C scanning electron microscope at 15 kV accelerating voltage. Differences between replicas were evident to the unaided eye and from Polaroid micrographs made of each replica at X30 to form a montage of the entire surface. Micrographs were also made at X1000 and X2000 to differentiate fine details of skin surface condition.

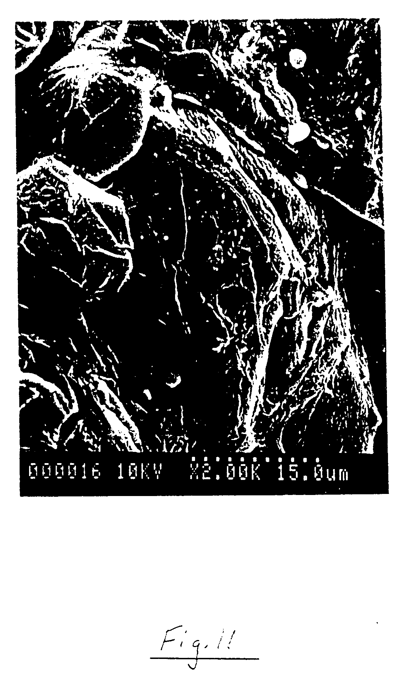

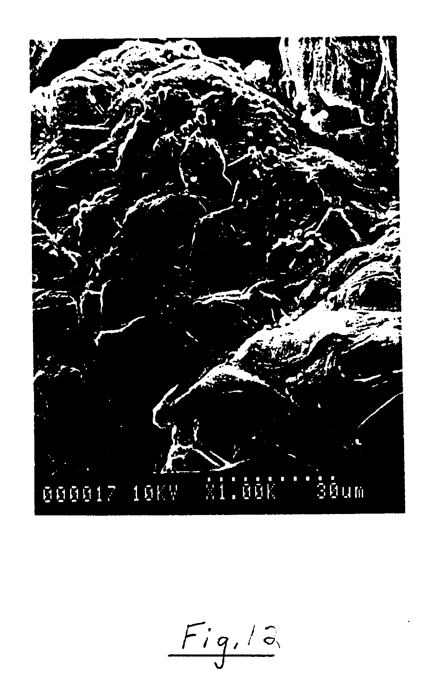

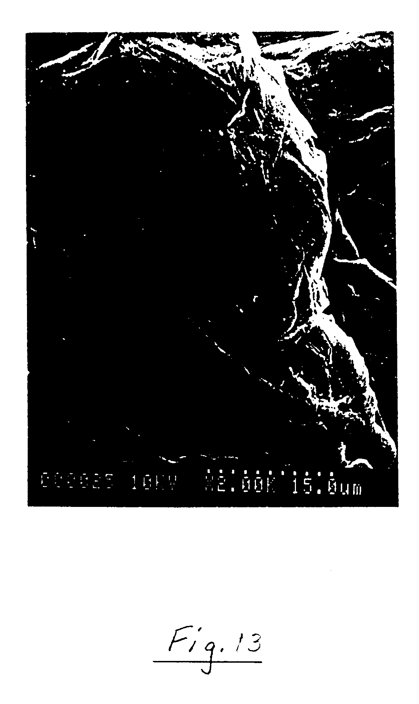

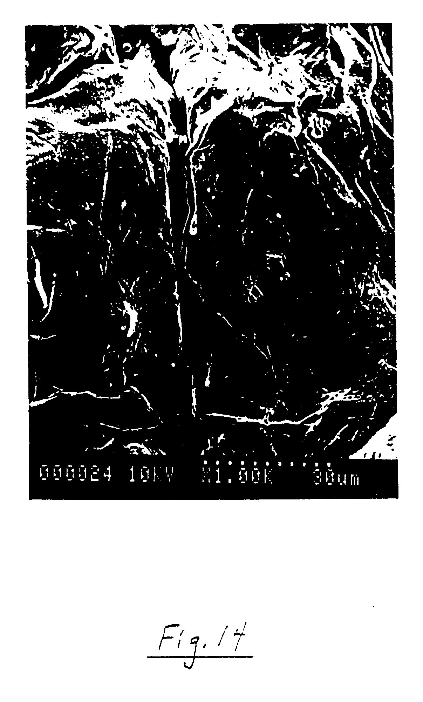

Figures 1 and 2 illustrate the skin surface condition of two of the control mice treated topically and intraperitoneally with a propylene glycol vehicle only. Figures 11 and 12 illustrate the skin condition of two of the experimental mice treated topically with 1α,25-dihydroxy-19-nor-vitamin D₃ whereas Figures 13 and 14 illustrate the skin condition of two of the experimental mice treated intraperitoneally with 1α,25-dihydroxy-19-nor-vitamin D₃.

The results of the above experiments were that the low magnification (X30) montages of the skin replicas could be ranked according to visible wrinkling and overall skin surface roughness. The best skin condition was exhibited by the mice which were treated with 1α,25-dihydroxy-19-nor-vitamin D₃. The controls could be placed alone in a rank as having the roughest skin (See Table 3).

Details of the skin surface such as stratum corneum desquamation, scaling, size and plumpness of skin divisions, were also visible on higher magnification (X1000 and X2000) micrographs and used to rank the replicas. The treated samples ranked the best and the controls ranked the worst. The top-ranked skin condition included all of the 19-nor samples. There was no discernible difference between topical and intraperitoneal 19-nor treated samples.

As a result of these experiments, it may be concluded that topical and intraperitoneal treatments of nude mice with 19-nor compounds produce visible changes in skin surface condition as seen on replicas. Treated mice skin showed less gross wrinkling and scaling and more smooth, plump, rounded skin microtopography than the skin of the respective controls. Topical treatment could not be differentiated from intraperitoneal treatment.

**TABLE 3**

| Response of Nude Mice to 19-Nor-1α,25-Dihydroxyvitamin D₃ | | | |
|---|---|---|---|
| Group | No. of Animals | Treatment | Observations |
| 1 | 4 | Vehicle (Propylene glycol) Topical | Skin remains scaly and wrinkled despite an initial moist appearance. |
| 2 | 3 | Propylene glycol vehicle - given intraperitoneally | Skin scaly and wrinkled. |
| 3 | 3 | 19-nor-1,25-(OH)₂D₃ topical in propylene glycol | Within 3 days, skin changed to a pinkish, less wrinkled and more plumpish appearance. Moist appearance continued throughout treatment. |
| 4 | 4 | 19-nor-1,25-(OH)₂ D₃ given intraperitoneally | After only 3 applications, skin acquired a pink, smoothe appearance with few wrinkles. Skin appeared moisturized. |
| I.P. = 50 ng 19-nor-1,25-(OH)₂D₃/0.05 ml propylene glycol given 3 times per week Topical = 100 ng 19-nor-1,25-(OH)₂D₃/0.02 ml applied 3 times per week. | | | |

The visible changes in skin surface condition (as shown in Figs. 11-14 versus Figs. 1-2) is in marked contrast with that of compositions containing ergocalciferol or cholecalciferol. Topical application of compositions containing ergocalciferol, for instance, were of low cosmetic efficacy and in fact resulted in decreased skin elasticity (See Table I in Dikstein et al Reissue No. 33,107). Moreover, since it is known that ergocalciferol and cholecalciferol are absorbed into the bloodstream through the skin, it is likely that doses of such compounds applied to large areas of the skin or applied chronically, even in the minimal active dose, cause systemic effects. Further, since the compounds in accordance with this invention have little or no classical vitamin D activities, no side effects were expected or observed.

### Cosmetic Efficacy of Secosterol Compounds

The cosmetic efficacy of compositions containing secosterol compounds used in this invention was determined by the following procedures:

Two treatment groups of six mice each were available with Group I being controls, and Group II being the treatment group with Homo-pregnacalciferol. Three mice in each group received either the control vehicle only or the Homo-pregnacalciferol treatment topically (t) and three mice in each group received either the control vehicle only or the Homo-pregnacalciferol treatment intraperitoneally (ip) three times a week for 5 weeks.

Replicas were made about 48 hours after the last treatment of the backs of 5 control animals and 4 experimental animals. Silflo™ silicone rubber was spread onto the rear half of each mouse back (anesthetized with diethyl ether) and allowed to polymerize for 5 to 7 minutes. These silicone rubber "negative" replicas were stored in glassine envelopes until polyethylene "positive" replicas were made. The procedures for preparing both the negative and positive replicas will hereinafter be described.

The nine positive replicas were coated with about 60 nm gold and examined in a JEOL JSM-35C scanning electron microscope at 15 kV accelerating voltage. Differences between replicas were evident to the unaided eye and from Polaroid micrographs made of each replica at X12 to form a montage of the entire surface. Micrographs were also made at X100 and X200 to differentiate fine details of skin surface condition.

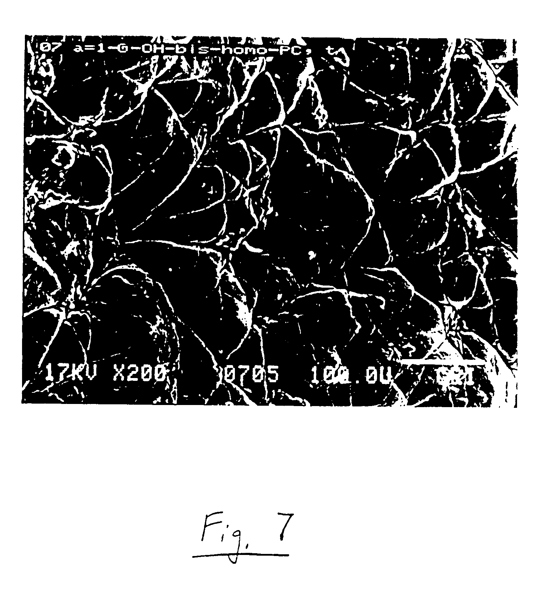

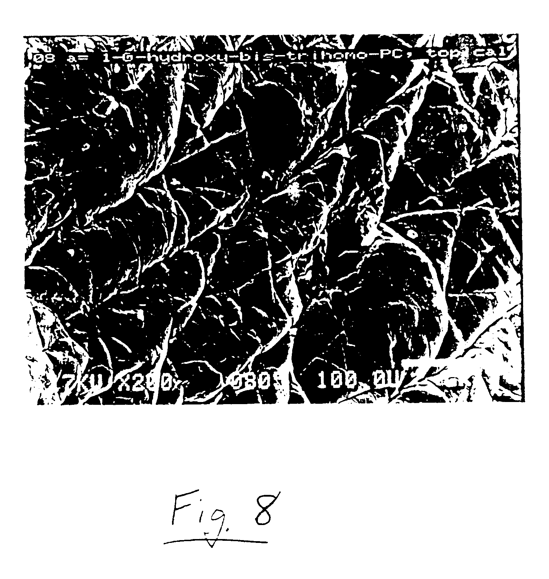

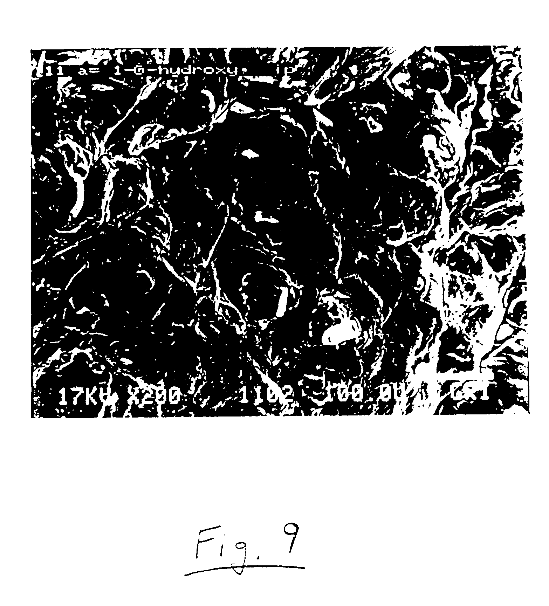

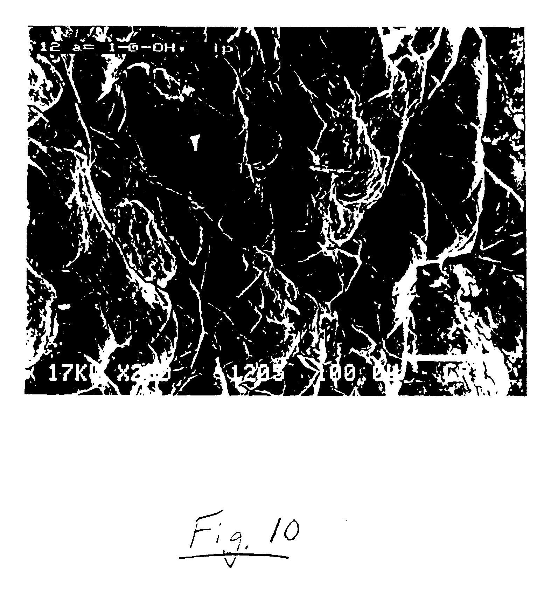

Figures 1 and 2 illustrate the skin surface condition of two of the control mice treated topically and intraperitoneally with a propylene glycol vehicle only. Figures 7 and 8 illustrate the skin condition of two of the experimental mice treated topically with 1α-hydroxy-bis-homo-pegnacalciferol (homo-pregna) whereas Figures 9 and 10 illustrate the skin condition of two of the experimental mice treated intraperitoneally with 1α-hydroxy-bis-homo-pregnacalciferol.

The results of the above experiments were that the low magnification (X12) montages of the nine skin replicas could be ranked into three groups according to visible wrinkling and overall skin surface roughness. The best skin condition (rank 1) was exhibited by sample #8 which was treated with Homo-pregna. The next best were #5, #6 and #7 which were all Homo-pregna treatments, but which could not be distinguished at this low magnification with #1 and #3 which were controls. Two controls could be placed alone in a third rank having the roughest skin (See Table 4).

Details of the skin surface such as stratum corneum desquamation, scaling, size and plumpness of skin divisions, were visible on higher magnification (X100 and X200) micrographs and used to rank the replicas from 1=best skin to 6=worst skin (Tables 4 and 5). The treated samples ranked 1 to 4, and the five controls ranked 5 and 6. The top-ranked four included all of the Homo-pregna samples (#5, #6, #7 and #8). There was no discernible difference between topical and intraperitoneal Homo-pregna treated samples, i.e. #5, #6, #7 and #8. As a result of these experiments, it may be concluded that topical and intraperitoneal treatments of nude mice with secosterol compounds produce visible changes in skin surface condition as seen on replicas. Treated samples showed less gross wrinkling and scaling and more smooth, plump, rounded skin microtopography than the respective controls. Topical treatment could not be differentiated from intraperitoneal treatment.

**TABLE 4**

| SEM OF MOUSE SKIN REPLICAS | | | |
|---|---|---|---|
| No. | Treatment | Rank*(X12) | Rank*(X100,200) |
| #1 | c-t control | 2 | 6 |
| #2 | c-t control | 3 | 6 |
| #3 | c-t control | 3 | 6 |
| #4 | c-ip control | 2 | 6 |
| #5 | a-t Homo-pregna | 2 | 2 |
| #6 | a-t Homo-pregna | 2 | 4 |
| #7 | a-ip Homo-pregna | 2 | 3 |
| #8 | a-ip Homo-pregna | 1 | 1 |
| #9 | c-ip control | 2 | 5 |

| | | | |
|---|---|---|---|
| *Scanning electron micrographs at X100 and X200 were judged for skin condition and ranked, with 1=best (smoothest, plumpest, least scaling) and 6=worst (roughest, most scaling). | | | |
| Treatments: a=Homo-pregnacalciferol | | | |
| c=Control, vehicle only | | | |
| t=topical, 80 ng Homo-pregnacalciferol/20»l propylene glycol applied 3 times per week | | | |
| ip=intraperitoneal, 25 ng Homo-pregnacalciferol/50»l propylene glycol given 3 times per week | | | |

**TABLE 5**

| RANKING OF MOUSE SKIN REPLICAS | | |
|---|---|---|
| No. | Treatment | Rank@X12) |
| 1 | #8 a-ip | 1 |
| 2 | #5 a-t | 2 |
| 3 | #7 a-ip | 2 |
| 4 | #6 a-t | 2 |
| 5 | #9 c-ip | 3 |
| 6 | #1 c-t | 2 |
| 6 | #2 c-t | 3 |
| 6 | #3 c-t | 2 |
| 6 | #4 c-ip | 2 |

| | | |
|---|---|---|
| *Scanning electron micrographs at X100 and X200 were judged for skin condition and ranked, with 1=best (smoothest, plumpest, least scaling) and 6=worst (roughest, most scaling). | | |
| @Photo montages at X12 magnification were made of each 25mm diameter replica and ranked for skin condition, with 1=best (smoothest) and 3=worst (roughest, more wrinkled). | | |
| Treatments: a=Homopregnacalciferol | | |
| c=Control, vehicle only | | |
| t=topical | | |
| ip=intraperitoneal | | |

The visible changes in skin surface condition (as shown in Figs. 7-10 versus Figs. 1-2) is in marked contrast with that of compositions containing ergocalciferol or cholecalciferol. Topical application of compositions containing ergocalciferol, for instance, were of low cosmetic efficacy and in fact resulted in decreased skin elasticity (See Table I in Dikstein et al Reissue No. 33,107). Moreover, since it is known that ergocalciferol and cholecalciferol are absorbed into the bloodstream through the skin, it is likely that doses of such compounds applied to large areas of the skin or applied chronically, even in the minimal active dose, cause systemic effects. Further, since the compounds in accordance with this invention have little or no classical vitamin D activities, no side effects were expected or observed.

The skin replication techniques utilized in preparing the "negative" and "positive" replicas in order to perform the above described experiments and the photomicrographs of Figs. 1-14 will now be described.
A. SILFLO NEGATIVE REPLICAS
   1. Mix Silflo well before dispensing. Put Silflo into plastic syringe, 5 or 10 ml size.
   2. Measure out 0.4 to 0.8 ml onto glassine paper or small weighing dish. The amount depends on the area to be replicated and the rate of polymerization desired.
   3. Add 1 drop thinner per 0.4 ml Silflo. (Steps 1-3 can be done in advance.)
   4. Place TCOM adhesive ring on skin site(s) to be replicated. (This ring was omitted on the mice.)
   5. Add 1 drop catalyst per 0.4 ml Silflo and start timer. These amounts can be adjusted if replica sets too fast or too slowly. Silflo should not stiffen until about 2 min. after catalyst addition and should set tack-free from about 3 to 3.5 min. after catalyst.
   6. Mix thoroughly with spatula tip for 20-25 sec.
   6a. To remove air bubbles, place Silflo dish into small vacuum desiccator and evacuate with mechanical pump until silicone rubber foams up once and collapses; remove at once from vacuum and apply to skin. (Elapsed time should be about 1 min. since addition of catalyst.)
   7. Spread Silflo mixture quickly onto skin site with spatula.
   8. Let Silflo set for a minimum of 5 minutes, without any movement of the subject. Check that the replica has polymerized before proceeding.
   9. Peel off the replica and place it in a dust and lint-free container.
B. POLYETHYLENE POSITIVE REPLICAS
   1. Allow Silflo replicas to polymerize completely at room temperature, usually overnight, but 6 hours is sufficient.
   2. Place replicas in a dish such as glass petri dish, aluminum weighing dish or on a metal tray. Place a shallow brass ring (or other retaining device which serves as a mold for the polyethylene) on the replica. The diameter of the ring mold will depend on the diameter of the SEM specimen carrier being used (usually 15mm or 25mm).
   3. Place replicas into 160°C to about 170°C oven for a short time, about 5 min. This heating of the replicas drives off moisture and other volatiles.
   4. Remove replicas from oven. Fill each brass ring mold with polyethylene pellets.
   5. Replace replicas in oven and heat until polyethylene has melted completely and covers the replica surface (about 15 min.).
   6. Turn oven off, open oven door and allow replicas to cool slowly. Too rapid cooling can cause cracks and artifacts in the polyethylene.
   7. When replicas have cooled almost to room temperature, they can be removed from the oven. Peel off the Silflo replica from the polyethylene replica, which remains within its metal ring.
   8. The hardened polyethylene replica can be sputter-coated with gold and examined in the SEM without removing it from its brass ring mold. The replica can also be pushed out of the ring mold and replaced in it again, if necessary.

## Claims

1. Use of a compound of formula I, II or III, for the manufacture of a cosmetic composition for the treatment of a cosmetic skin condition wherein formula I is: 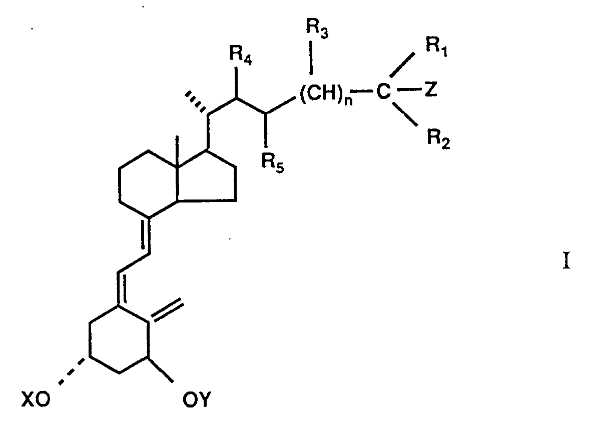 where R₄ and R₅ are each a hydrogen atom or when taken together R₄ and R₅ form a single bond or a double bond, Z is a hydrogen atom, a hydroxy or protected-hydroxy group, R₃ is a hydroxy, protected-hydroxy or an alkyl group, X and Y which may be the same or different, are a hydrogen atom or a hydroxy-protecting group, R₁ is a -(CH₂)_{q}-H and R₂ is a -(CH₂)ₚ-H, and n, q and p are independently integers of 1 to 5 or R₁ and R₂ when taken together also represent the group -(CH₂)ₘ- where m is an integer of 2 to 5, with the proviso that at least one of n, q and p is greater than 1;
wherein formula II is: 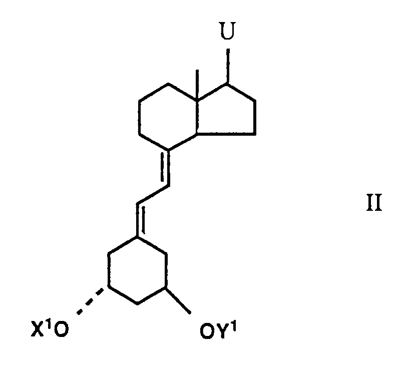 wherein X¹ and Y² are each a hydrogen atom, an acyl, alkylsilyl or alkoxyalkyl group, and U is a hydrogen atom or an alkyl, hydroxyalkyl or fluoroalkyl group or a side chain of the formula 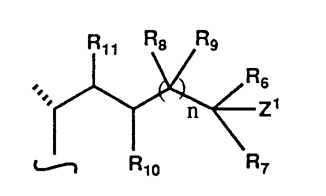 wherein Z¹ is a hydrogen atom or a hydroxy or O-acyl group, R₆ and R₇ which may be the same or different are an alkyl, hydroxyalkyl or fluoroalkyl group, or when taken together are -- (CH₂)ₘ -- group, where m is an integer of 2 to 5, R₈ is a hydrogen atom, a fluorine atom, or a hydroxy, O-acyl, alkyl, hydroxyalkyl or fluoroalkyl group, R₉ is a hydrogen atom, a fluorine atom or an alkyl, hydroxyalkyl or fluoroalkyl group, or R₈ and R₉ taken together represent double-bonded oxygen or double-bonded carbon, R₁₀ and R₁₁, which may be the same or different, are a hydrogen atom, a fluorine atom, a hydroxy , O-acyl or alkyl group, or R₁₀ and R₁₁ taken together form a bond or a double bond, and n is an integer of 1 to 5 and the carbon at any one of positions 20, 22, or 23 in the side chain may be replaced by an O, S, or N atom; and wherein formula III is: 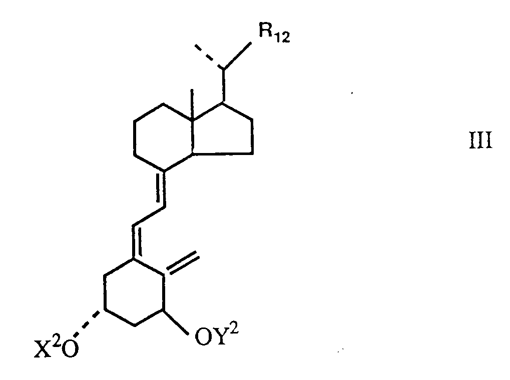 where R₁₂ is a hydrogen atom or a methyl, ethyl or propyl group and X² and Y² which may be the same or different are a hydrogen atom, an acyl group, or a hydroxy-protecting group.

2. Use according to claim 1 of a compound of formula 1.

3. Use according to claim 1 of a compound of formula II.

4. Use according to claim 1 of a compound of formula III.

5. Use according to any one of claims 1 to 4 wherein the composition contains 0.001 »g to 10.0»g per gram of the compound.

6. Use according to any one of claims 1 to 5 wherein the composition is administered to a patient by topical, oral or parenteral means.

7. Use according to any one of the preceding claims wherein the composition is used to provide an amount of the said compound of 0.1»g/day to 25 »g/day.

8. A method of counteracting a cosmetic skin condition, which comprises applying to the skin a compound of formula I, II or III as defined in claim 1.

9. A method according to claim 8 which comprises applying a compound of formula I.

10. A method according to claim 8 which comprises applying a compound of formula II.

11. A method according to claim 8 which comprises applying a compound of formula III.

12. A method according to any one of claims 8 to 11 wherein the compound is used in an amount of 0.1 »g/day to 25 »g/day.

13. A method according to any one of claims 8 to 12 wherein the compound is applied as a composition which contains 0.001 »g to 10 »g per gram of compound.

14. A method according to any one of claims 8 to 13 for counteracting wrinkles in the skin and/or a lack of adequate skin firmness.

## Patentansprüche

1. Verwendung einer Verbindung der Formel I, II oder III zur Herstellung einer kosmetischen Zusammensetzung zur Behandlung einer kosmetischen Hautbeschaffenheit, wobei Formel I bedeutet: 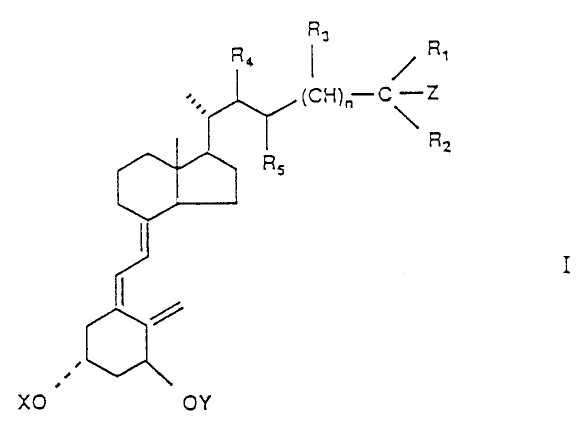 worin R₄ und R₅ jeweils ein Wasserstoffatom bedeuten oder R₄ und R₅ zusammen eine Einfachbindung oder eine Doppelbindung bilden, Z ein Wasserstoffatom, eine Hydroxy- oder geschützte Hydroxygruppe ist, R₃ eine Hydroxy-, geschützte Hydroxy- oder eine Alkylgruppe ist, X und Y, die gleich oder verschieden sein können, ein Wasserstoffatom oder eine Hydroxy-Schutzgruppe sind, R₁ -(CH₂)_{q}-H und R₂ -(CH₂)ₚ-H ist, und n, q und p unabhängig voneinander ganze Zahlen von 1 bis 5 sind, oder R₁ und R₂ zusammen auch die Gruppe -(CH₂)ₘ- darstellen, worin m eine ganze Zahl von 2 bis 5 ist, mit der Maßnahme, daß mindestens eine der Bedeutungen n, q und p größer als 1 ist;
Formel II bedeutet: 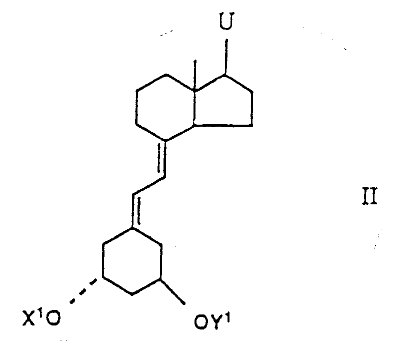 worin X¹ und Y¹ jeweils ein Wasserstoffatom, eine Acyl-, Alkylsilyl- oder Alkoxyalkylgruppe bedeuten, und U ein Wasserstoffatom oder eine Alkyl-, Hydroxyalkyl oder Fluoralkylgruppe bedeutet, oder eine Seitenkette der Formel 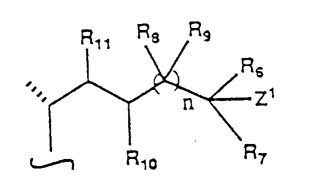 worin Z¹ ein Wasserstoffatom oder eine Hydroxy- oder O-Acylgruppe ist, R₆ und R₇, die gleich oder verschieden sein können, eine Alkyl-, Hydroxyalkyl- oder Fluoralkylgruppe bedeuten, oder zusammen eine -(CH₂)ₘ-Gruppe darstellen, worin m eine ganze Zahl von 2 bis 5 ist, R₈ ein Wasserstoffatom, ein Fluoratom, oder eine Hydroxy-, O-Acyl, Alkyl-, Hydroxyalkyl- oder Fluoralkylgruppe ist, R₉ ein Wasserstoffatom, ein Fluoratom oder eine Alkyl-, Hydroxyalkyl- oder Fluoralkylgruppe ist, oder R₈ und R₉ zusammen doppelt gebundenen Sauerstoff oder doppelt gebundenen Kohlenstoff darstellen, R₁₀ und R₁₁, die gleich oder verschieden sein können, ein Wasserstoffatom, ein Fluoratom, eine Hydroxy-, O-Acyl- oder Alkylgruppe sind, oder R₁₀ und R₁₁ zusammen eine Einfach- oder Doppelbindung bilden, und n eine ganze Zahl von 1 bis 5 ist, und das Kohlenstoff an einer der Positionen 20, 22 oder 23 in der Seitenkette durch ein O, S oder N-Atom ersetzt sein kann; und Formel III bedeutet: 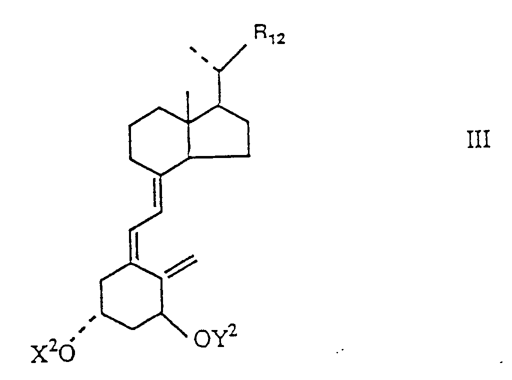 worin R₁₂ ein Wasserstoffatom oder eine Methyl-, Ethyl oder Propylgruppe ist und X² und Y², die gleich oder verschieden sein können, ein Wasserstoffatom, eine Acylgruppe oder eine Hydroxy-Schutzgruppe sind.

2. Verwendung nach Anspruch 1, dadurch gekennzeichnet, daß man eine Verbindung der Formel I verwendet.

3. Verwendung nach Anspruch 1, dadurch gekennzeichnet, daß man eine Verbindung der Formel II verwendet.

4. Verwendung nach Anspruch 1, dadurch gekennzeichnet, daß man eine Verbindung der Formel III verwendet.

5. Verwendung nach einem der Ansprüche 1 bis 4, dadurch gekennzeichnet, daß die Zusammensetzung pro Gramm 0.001 »g bis 10.0 »g der Verbindung enthält.

6. Verwendung nach einem der Ansprüche 1 bis 5, dadurch gekennzeichnet, daß die Zusammensetzung einem Patienten auf topische, orale oder parenterale Weise verabreicht wird.

7. Verwendung nach einem der vorhergehenden Ansprüche, dadurch gekennzeichnet, daß die Zusammensetzung so verwendet wird, um die Verbindung in einer Menge von 0.1 »g/Tag bis 25 »g/Tag bereitzustellen.

8. Verfahren zur Behandlung einer kosmetischen Hautbeschaffenheit, dadurch gekennzeichnet, daß man auf die Haut eine Verbindung der Formel I, II oder III nach Anspruch 1 appliziert.

9. Verfahren nach Anspruch 8, dadurch gekennzeichnet, daß man eine Verbindung der Formel I appliziert.

10. Verfahren nach Anspruch 8, dadurch gekennzeichnet, daß man eine Verbindung der Formel II appliziert.

11. Verfahren nach Anspruch 8, dadurch gekennzeichnet, daß man eine Verbindung der Formel III appliziert.

12. Verfahren nach einem der Ansprüche 8 bis 11, dadurch gekennzeichnet, daß die Verbindung in einer Menge von 0.1 »g/Tag bis 25 »g/Tag verwendet wird.

13. Verfahren nach einem der Ansprüche 8 bis 12, dadurch gekennzeichnet, daß die Verbindung in Form einer Zusammensetzung appliziert wird, die pro Gramm 0.001 »g bis 10 »g der Verbindung enthält.

14. Verfahren nach einem der Ansprüche 8 bis 13 zur Vorbeugung oder Behandlung von Hautfalten und/oder einer nicht ausreichend straffen Haut.

## Revendications

1. Emploi d'un composé de formule I, II ou III pour la préparation d'une composition cosmétique destinée au traitement d'un état cosmétologique de la peau,
la formule I étant la suivante : 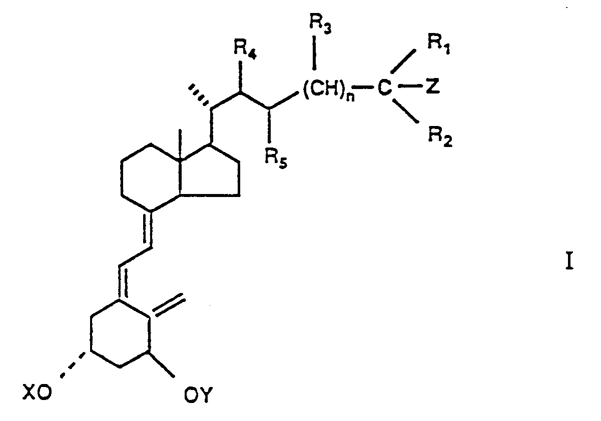 dans laquelle R₄ et R₅ représentent chacun un atome d'hydrogène ou forment ensemble une simple liaison ou une double liaison, Z représente un atome d'hydrogène, un groupe hydroxy ou un groupe hydroxy protégé, R₃ représente un groupe hydroxy, un groupe hydroxy protégé ou un groupe alkyle, X et Y, qui peuvent être identiques ou différents, représentent chacun un atome d'hydrogène ou un groupe hydroxy-protecteur, R₁ représente un groupe de formule -(CH₂)_{q}-H et R₂ représente un groupe de formule -(CH₂)ₚ-H, et n, p et q représentent indépendamment des nombres entiers valant de 1 à 5, ou bien R₁ et R₂ représentent ensemble un groupe de formule -(CH₂)ₘ- où m représente un nombre entier valant de 2 à 5, sous réserve qu'au moins l'un des indices n, p et q soit supérieur à 1;
la formule II étant la suivante : 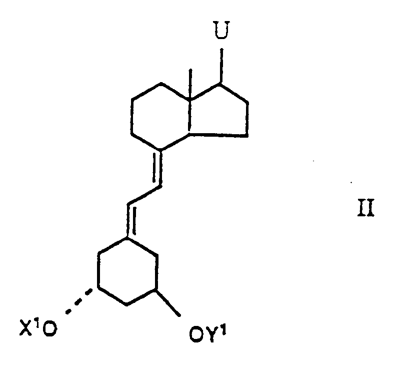 dans laquelle X¹ et Y² représentent chacun un atome d'hydrogène ou un groupe acyle, alkylsilyle ou alcoxyalkyle, et U représente un atome d'hydrogène ou un groupe alkyle, hydroxyalkyle ou fluoroalkyle, ou encore une chaîne latérale de formule 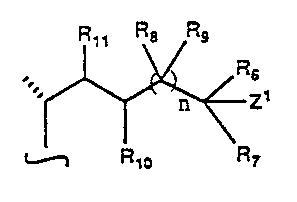 dans laquelle Z¹ représente un atome d'hydrogène ou un groupe hydroxy ou O-acyle, R₆ et R₇, qui peuvent être identiques ou différents, représentent chacun un groupe alkyle, hydroxyalkyle ou fluoroalkyle, ou bien représentent ensemble un groupe de formule -(CH₂)ₘ- où m représente un nombre entier valant de 2 à 5, R₈ représente un atome d'hydrogène ou de fluor ou un groupe hydroxy, O-acyle, alkyle, hydroxyalkyle ou fluoroalkyle, R₉ représente un atome d'hydrogène ou de fluor ou un groupe alkyle, hydroxyalkyle ou fluoroalkyle, ou bien R₈ et R₉ représentent ensemble un atome d'oxygène ou de carbone lié par une double liaison, R₁₀ et R₁₁, qui peuvent être identiques ou différents, représentent chacun un atome d'hydrogène ou de fluor ou un groupe hydroxy, O-acyle ou alkyle, ou bien R₁₀ et R₁₁ représentent ensemble une liaison ou une double liaison, et n représente un nombre entier valant de 1 à 5, et l'atome de carbone situé en n'importe laquelle des positions 20, 22 et 23 de la chaîne latérale peut être remplacé par un atome d'oxygène, de soufre ou d'azote;
et la formule III étant la suivante : 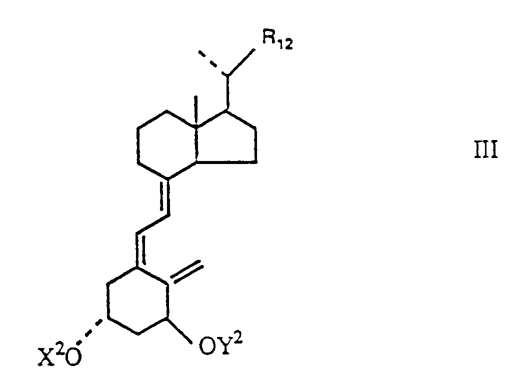 dans laquelle R₁₂ représente un atome d'hydrogène ou un groupe méthyle, éthyle ou propyle, et X² et Y², qui peuvent être identiques ou différents, représentent chacun un atome d'hydrogène, un groupe acyle ou un groupe hydroxy-protecteur.

2. Emploi, conforme à la revendication 1, d'un composé de formule I.

3. Emploi, conforme à la revendication 1, d'un composé de formule II.

4. Emploi, conforme à la revendication 1, d'un composé de formule III.

5. Emploi conforme à l'une des revendications 1 à 4, dans lequel la composition contient, par gramme, de 0,001 »g à 10,0 »g de composé.

6. Emploi conforme à l'une des revendications 1 à 5, dans lequel la composition est administrée à un patient en application locale, par voie orale ou par voie parentérale.

7. Emploi conforme à l'une des revendications précédentes, dans lequel la composition est utilisée de façon à fournir de 0,1 »g/jour à 25 »g/jour dudit composé.

8. Procédé pour contrecarrer un état cosmétologique de la peau, qui comporte le fait d'appliquer sur la peau un composé de formule I, II ou III, définie dans la revendication 1.

9. Procédé conforme à la revendication 8, qui comporte l'application d'un composé de formule I.

10. Procédé conforme à la revendication 8, qui comporte l'application d'un composé de formule II.

11. Procédé conforme à la revendication 8, qui comporte l'application d'un composé de formule III.

12. Procédé conforme à l'une des revendications 8 à 11, dans lequel on emploie le composé à raison de 0,1 »g/jour à 25 »g/jour.

13. Procédé conforme à l'une des revendications 8 à 12, dans lequel on applique le composé sous la forme d'une composition qui contient, par gramme, de 0,001 »g à 10 »g de composé.

14. Procédé conforme à l'une des revendications 8 à 13, pour contrecarrer des rides de la peau et/ou un manque de fermeté adéquate de la peau.
